# EUROPÄISCHE PATENTSCHRIFT

(11) **EP 1 544 596 B1**
(45) Veröffentlichungstag und Bekanntmachung des Hinweises auf die Patenterteilung: **23.11.2016**
(21) Anmeldenummer: 04028644.5
(22) Anmeldetag: 03.12.2004
(51) Int. Cl.: G01N 11/10, G01N 11/16, G01N 33/49

(54) **Verfahren und Vorrichtung zur Bestimmung der Viskosität**
Method and device for determining viscosity
Méthode et appareil de détermination de la viscosité

(30) Priorität: 17.12.2003 DE 10359438; 25.02.2004 DE 4009089
(43) Veröffentlichungstag der Anmeldung: 22.06.2005
(73) Patentinhaber: Boehringer Ingelheim microParts GmbH, 44227 Dortmund (DE)
(72) Erfinder: Kurowski, Dirk, Dr., 58332 Schwelm (DE); Schön, Christian, 44229 Dortmund (DE); Peters, Ralf-Peter, Dr., 51467 Bergisch-Gladbach (DE); Bartos, Holger, Dr., 44287 Dortmund (DE); Yu, Ying, Dr., 33275 Dortmund (DE)
(74) Vertreter: Von Rohr Patentanwälte Partnerschaft mbB

(56) Entgegenhaltungen:
- WO-A-01/86255
- DE-A1- 19 745 807
- US-A- 3 967 934
- US-A- 4 864 849
- US-A- 5 110 727
- US-A- 5 394 739
- US-B2- 6 591 664
- SIMON ET AL.: "diamagnetically stabilized magnet levitation" 29. März 2001 (2001-03-29), XP002300363 Gefunden im Internet: URL:WWW.PHYSICS.UCLA.EDU/MARTY/DIAMAG/LEVI DOT.PDF>
- PATENT ABSTRACTS OF JAPAN Bd. 1996, Nr. 11, 29. November 1996 (1996-11-29) & JP 08 178823 A (YKK KK), 12. Juli 1996 (1996-07-12)
- ZIEMANN F ET AL: "Local measurements of viscoelastic moduli of entangled actin networks using an oscillating magnetic bead micro-rheometer", BIOPHYSICAL JOURNAL, CELL PRESS, US, vol. 66, no. 6, 1 June 1994 (1994-06-01), pages 2210-2216, XP008122046, ISSN: 0006-3495
- Andreas R. Bausch ET AL: "Measurement of Local Viscoelasticity and Forces in Living Cells by Magnetic Tweezers", Biophysical Journal, vol. 76, no. 1, 1 January 1999 (1999-01-01), pages 573-579, XP055199761, ISSN: 0006-3495, DOI: 10.1016/S0006-3495(99)77225-5

## Beschreibung

Die vorliegende Erfindung betrifft ein Verfahren gemäß dem Oberbegriff des Patentanspruchs 1 und eine Vorrichtung gemäß dem Oberbegriff des Patentanspruchs 21 zur Bestimmung der Viskosität oder. einer damit zusammenhängenden Größe eines Fluids oder von magnetische Partikeln im Fluid, insbesondere eines Mikrofluids, sowie Verwendungen eines derartigen Verfahrens oder einer derartigen Vorrichtung.

Die US 3,967,934 A offenbart ein System und Verfahren zur Bestimmung der Gerinnungszeit von Blut. Ein Testrohr mit dem Blut wird auf- und abbewegt, wobei eine Metallkugel im Blut innerhalb des Testrohrs mittels eines stationären Magnetfelds in einer vertikalen Lage gehalten wird. Beim Auf- und Abbewegen des Testrohrs strömt das Blut um die Metallkugel herum. Wenn die Gerinnung (Koagulation) des Bluts erfolgt wird die Metallkugel aus ihrer definierten Vertikallage ausgelenkt. Durch diese Auslenkung wird eine Lichtschranke freigegeben und dies als Gerinnungszeit erfaßt. Das System ist sehr aufwendig und insbesondere sind bewegte Teile erforderlich. Das Verfahren eignet sich nicht für die Mikrofluidik, also Proben mit geringem Volumen, insbesondere im Bereich von einem Milliliter oder weniger. Auch sind keine Informationen über den Verlauf der Gerinnung erhältlich.

Die JP 8-178823 A offenbart ein Verfahren und eine Vorrichtung zur Messung der Viskosität von viskosem Material. Als Meßelemente werden feine, weichmagnetische Partikel verwendet, die durch ein externes stationäres Magnetfeld in dem viskosen Material, zum Beispiel Silikon oder Acrylharzin, in eine Richtung bewegt werden. Mittels eines beispielsweise auf dem magnetoresistiven Effekt beruhenden Sensors wird die Ankunft der sich bewegenden magnetischen Partikel detektiert und die Geschwindigkeit und Zeit der Bewegung zur Bestimmung der Viskosität gemessen. Gegenüber ansonsten nur durch Gravitation absinkenden Meßelementen kann eine wesentlich geringere Meßzeit von etwa zwei bis fünf Minuten erreicht werden. Bei dem bekannten Verfahren ist nachteilig, daß mehrere Versuche mit jeweils erforderlicher Zugabe der magnetischen Partikel notwendig sind, um eine sinnvolle Meßgenauigkeit erreichen zu können. Das Verfahren ist dementsprechend aufwendig und langwierig. Das Verfahren eignet sich nicht für die Mikrofluidik, also Proben mit geringem Volumen, insbesondere im Bereich von einem Milliliter oder weniger. Auch sind keine Informationen über den Verlauf der Gerinnung erhältlich.

Die WO 01/86255 A1 betrifft eine Vorrichtung zur Bestimmung der Viskosität eines Fluids oder Gels. Hierbei wird ein Partikel mittels einer Kraft manipuliert und die Phasenverschiebung zwischen der anregenden Kraft und der Bewegungspartikels optisch beobachtet und hieraus eine lokale Viskosität bestimmt.

Die DE 197 45 807 A1 betrifft ein Rheometer zur Bestimmung viskoelastischer Eigenschaften von lebenden Zellen. Magnetische Partikel werden magnetisch in Schwingung versetzt und mit einem Mikroskop beobachtet. Auch hier wird die Phasenverschiebung zwischen Partikelbewegung und anregender Kraft beobachtet, um Rückschlüsse auf die viskoelastischen Eigenschaften der Zellen zu erhalten. Zudem wird erwähnt, dass zur Detektion der Antwortbewegung alternativ ein in einer Messspule induzierter Strom verwendet werden kann.

Der vorliegenden Erfindung liegt die Aufgabe zugrunde, ein Verfahren und eine Vorrichtung zur Bestimmung der Viskosität oder einer damit zusammenhängenden Größe eines Fluids oder von mikroskopischen Partikeln im Fluid, insbesondere eines Mikrofluids, sowie Verwendungen dieses Verfahrens und dieser Vorrichtung anzugeben, wobei ein einfacher, kompakter Aufbau und/oder eine genaue Bestimmung der Viskosität oder einer damit zusammenhängenden Größe ermöglicht wird bzw. werden, insbesondere wobei die Viskosität bzw. die damit zusammenhängende Größe fortlaufend bestimmt werden kann bzw. können.

Die obige Aufgabe wird durch ein Verfahren gemäß Anspruch 1, eine Vorrichtung gemäß Anspruch 21 oder eine Verwendung gemäß Anspruch 44, 45 oder 46 gelöst. Vorteilhafte Weiterbildungen sind Gegenstand der Unteransprüche.

Eine grundlegende Idee der vorliegenden Erfindung liegt darin, magnetische Partikel im Fluid durch zeitliche Variation eines Magnetfelds in Schwingung zu versetzen, so daß der von dem Fluid vollständig umgebene Partikel führungsfrei, also ohne mechanische Führung im Fluid oder in einer Meßkammer, frei im Fluid schwimmt, wobei die Amplitude und/oder Phase der Schwingung zumindest indirekt gemessen wird bzw. werden, um daraus die Viskosität oder eine damit zusammenhängende Größe, wie die Reynolds-Zahl, die Strouhal-Zahl oder dergleichen, zu bestimmen. Dies ermöglicht eine einfache und genaue Bestimmung der Viskosität bzw, der damit zusammenhängenden Größe, wobei insbesondere ein kompakter und einfacher Aufbau, vorzugsweise ohne mechanisch bewegte Teile, und eine fortlaufende Bestimmung der Viskosität mit hoher Genauigkeit ermöglicht werden. Als "Phase der Schwingung" wird hier kurz gefaßt die Phasenverschiebung der Schwingung der Partikel gegenüber dem zeitlich variierenden (anregenden) Magnetfeld bezeichnet.

Die Viskosität muß nicht unmittelbar oder absolut bestimmt werden. Vielmehr kann es bedarfsweise genügen, die Viskosität nur relativ zu bestimmen oder eine Größe zu bestimmen, die mit der Viskosität in einem funktionellen, insbesondere eindeutigen Zusammenhang steht bzw. von der Viskosität abhängt.

Der Begriff "Viskosität" ist bei der vorliegenden Erfindung in einem engeren Sinne als innere Reibung oder Möglichkeit einer Spannungsaufnahme bei Verformung eines Fluids, insbesondere einer Flüssigkeit, zu verstehen. In einem weiteren Sinne ist bzw. sind unter "Viskosität" bzw. einer damit zusammenhängenden Größe auch eine Veränderung von Eigenschaften des gegebenenfalls inhomogenen Fluids, insbesondere durch Koagulation, Quellen von Bestandteilen oder dergleichen, und/oder Eigenschaften der Partikel, insbesondere deren Masse, magnetisches Moment oder Beweglichkeit oder die Dämpfung der Partikelschwingung, im Fluid, beispielsweise durch Anlagerung oder Ablösen von Atomen oder Molekülen an bzw. von den Partikeln oder dergleichen, oder diesbezügliche Änderungen zu verstehen.

Das vorschlagsgemäße Verfahren und die vorschlagsgemäße Vorrichtung können insbesondere zu nichttherapeutischen Zwecken zur Bestimmung der Koagulationsfähigkeit (Gerinnungsfähigkeit) von Blut oder Blutplasma, zur Bestimmung einer Anlagerung von Atomen oder Molekülen an die magnetischen Partikel oder Ablösung davon oder zur Bestimmung einer Glucosekonzentration, wobei die Viskosität eines Fluids bestimmt wird, das beispielsweise über eine für Glucose durchlässige Membran mit Blut in Verbindung steht und dessen Viskosität von der Glucosekonzentration abhängt, verwendet werden.

Weitere Vorteile, Merkmale, Eigenschaften und Aspekte der vorliegenden Erfindung ergeben sich aus der folgenden Beschreibung bevorzugter Ausführungsformen anhand der Zeichnung. Es zeigt:
- Fig. 1: eine schematische, blockschaltbildartige Darstellung einer vorschlagsgemäßen Vorrichtung gemäß einer ersten Ausführungsform;
- Fig. 2: ein schematisches Phasendiagramm von variierenden Magnetfeldern und einer auf magnetische Partikel wirkenden Kraft;
- Fig. 3: eine schematische, blockschaltbildartige Darstellung einer Vorrichtung, welche nicht Teil der Erfindung ist.
- Fig. 4: eine schematische Schnittdarstellung einer vorschlagsgemäßen Vorrichtung gemäß einer dritten Ausführungsform; und
- Fig. 5: eine schematische Darstellung einer vorschlagsgemäßen Vorrichtung gemäß einer vierten Ausführungsform.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

In einem inhomogenen Magnetfeld erfährt ein magnetischer Dipol eine Kraft entlang eines Feldgradienten. Dementsprechend können magnetische Partikel innerhalb eines Fluids mittels eines inhomogenen Magnetfelds bewegt werden. Durch zeitliche Variation des Magnetfelds werden die magnetischen Partikel vorschlagsgemäß in Schwingung versetzt. Auf diesem Prinzip beruhen die vorschlagsgemäße Vorrichtung und das vorschlagsgemäße Verfahren.

Vorschlagsgemäß erfolgt dann eine zumindest indirekte Messung der Amplitude und/oder Phase der Schwingung der Partikel. Diese hängen von der Dämpfung der Partikelschwingung ab und stellen daher ein Maß für die Viskosität oder sonstige Eigenschaften des Fluids und/oder der Partikel dar. Dementsprechend kann aus der Amplitude und/oder Phase die Viskosität - insbesondere im oben genannten, weiten Sinn - oder eine damit zusammenhängende Größe des Fluids bzw. der Partikel bestimmt werden.

Die Viskosität muß nicht unmittelbar oder absolut bestimmt oder ausgegeben werden. Vielmehr kann es genügen, die Viskosität nur relativ zu bestimmen oder eine Größe zu bestimmen, die mit der Viskosität in einem funktionellen, insbesondere eineindeutigen Zusammenhang steht bzw. von der Viskosität abhängt.

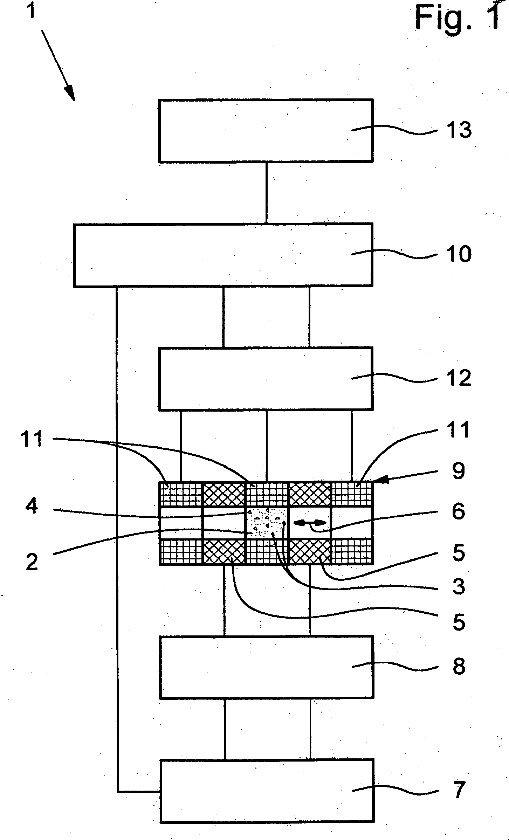

Fig. 1 zeigt in schematischer Darstellung eine vorschlagsgemäße Vorrichtung 1 gemäß einer ersten Ausführungsform zur Bestimmung der Viskosität oder einer damit zusammenhängenden Größe eines Fluids 2.

Bei dem Fluid 2 handelt es sich vorzugsweise um eine Flüssigkeit, insbesondere für biologische oder chemische Untersuchungen oder Diagnostik.

Das Volumen des zu messenden Fluids kann sehr gering sein und liegt vorzugsweise nur im µl-Bereich. Insbesondere handelt es sich also um ein sogenanntes Mikrofluid.

Das Fluid 2 weist mindestens einen magnetischen Partikel 3, insbesondere mehrere magnetische Partikel 3, auf. Nachfolgend werden immer mehrere magnetische Partikel 3 beschrieben. Alle diesbezüglichen Ausführungen gelten generell entsprechend, auch wenn nur ein einziger Partikel 3 im Fluid 2 vorgesehen ist.

Vorzugsweise sind die magnetischen Partikel 3 nur dem erforderlichen Meßvolumen des Fluids 2 bzw. nur in einem Raumbereich oder örtlichen Meßbereich zugesetzt, so daß die erforderliche Menge an magnetischen Partikeln 3 gering ist. Jedoch können die Partikel 3 auch im gesamten Fluid 2 verteilt sein.

Die Partikel 3 enthalten vorzugsweise Eisenoxid, insbesondere Magnetit oder sonstiges Ferrit. Jedoch können die Partikel 3 auch sonstige geeignete magnetische Materialien enthalten.

Die Partikel 3 sind vorzugsweise superparamagnetisch. Dies bedeutet, daß sie eine zu Ferromagneten korrespondierende Magnetisierungskurve, jedoch keine Remanenz aufweisen.

Grundsätzlich können jedoch auch sonstige magnetische, insbesondere paramagnetische oder ferromagnetische Partikel 3 eingesetzt werden.

Die magnetischen Partikel 3 weisen vorzugsweise eine Hülle, insbesondere aus Kunststoff, auf.

Die Partikel 3 sind vorzugsweise zumindest im wesentlichen kornförmig, kugelförmig und/oder ellipsoidförmig ausgebildet

Die Partikel 3 sind vorzugsweise zumindest im wesentlichen kugelförmig ausgebildet. Der mittlere Durchmesser der Partikel 3 beträgt vorzugsweise 20 nm bis 1000 µm, insbesondere etwa 100 nm bis etwa 500 µm und ganz besonderes bevorzugt etwa 0,5 µm bis etwa 100 µm.

Vorzugsweise werden Partikel 3 mit zumindest im wesentlichen einheitlicher Form und/oder Größe bzw. gleichem mittleren Durchmesser verwendet.

Die Partikel 3 sind im Fluid 2 frei schwimmend, also insbesondere ohne mechanische Führung im Fluid 2 angeordnet. Die Dichte der Partikel 3 entspricht vorzugsweise zumindest im wesentlichen der Dichte des Fluids 2 oder ist vorzugsweise größer als die Dichte des Fluids 2. Das Fluid 2 umgibt die Partikel 3 - zumindest die für die Messung relevanten Partikel 3 - ständig vollständig.

Die Vorrichtung 1 weist eine Meßkammer 4 zur Aufnahme des Fluids 2 und der Partikel 3 auf. Die Vorrichtung 1 weist weiter zwei Spulen 5, zur Erzeugung eines zeitlich variierenden Magnetfelds 6 in der Meßkammer 4 oder in einem Meßbereich davon auf. Insbesondere ist die Meßkammer 4 zwischen den beabstandeten Spulen 5 angeordnet und/oder von diesen umgeben.

Eine Steuereinrichtung 7 - insbesondere mit zwei Oszillatoren oder Funktionsgeneratoren, deren Amplituden, Frequenzen, Phasen und/oder Offset vorzugsweise einstellbar ist bzw. sind - und ggf. ein dazwischen geschalteter Verstärker 8 sind den beiden Spulen 5 zugeordnet, um sinusförmige, um 90° phasenversetzte Magnetfelder mittels der Spulen 5 zu erzeugen.

Die von den Spulen 5 erzeugten Magnetfelder addieren sich zu dem zeitlich variierenden, auf die Partikel 3 wirkenden, inhomogenen Magnetfeld 6, vorzugsweise einem Wechselfeld. Mittels des zeitlich variierenden Magnetfelds 6 werden die Partikel 3 in Schwingung versetzt, und zwar in hin- und hergehende, also translatorische Bewegungen.

Die Vorrichtung 1 ist derart ausgebildet bzw. die Ansteuerung der das Magnetfeld 6 erzeugenden Spulen 5 erfolgt derart, daß die Partikel 3 eine erzwungene, sinusförmige Schwingung ausführen.

Die Vorrichtung 1 weist eine Sensoreinrichtung 9 und eine Meßeinrichtung 10 auf, um die Bewegung - also Schwingung - der Partikel 3 im Fluid 2 magnetische zu erfassen und die Amplitude und/oder Phase der Schwingung zu messen.

Beim Darstellungsbeispiel weist die Sensoreinrichtung 9 mindestens eine Meßspule 11, insbesondere mehrere Meßspulen 11 auf. Insbesondere sind insgesamt drei Meßspulen 11 vorgesehen, wobei diese vorzugsweise paarweise an gegenüberliegenden Enden einer Spule 5 angeordnet und entgegengesetzt gewickelt sind. Dies dient einer Kompensation der von den Spulen 5 erzeugten Magnetfelder, so daß die Schwingung der Partikel 3 leichter erfaßbar bzw. meßbar ist.

Beim Darstellungsbeispiel wird die mittlere Meßspule 11 für beide Spulenpaare, bei der Darstellung gemäß Fig. 1 also sowohl für das rechte als auch für das linke Spulenpaar eingesetzt. Dies spart eine vierte Meßspule 11. Jedoch kann grundsätzlich auch eine vierte Meßspule 11 zur Bildung zweier getrennter Meßspulenpaare vorgesehen sein.

Bei Detektion der Schwingung mittels Spulen 11 wird insbesondere die induzierte Spannung gemessen, die von der Frequenz, der Amplitude und dem magnetischen Moment der Partikel 3 abhängt.

Die Signale der Meßspulen 11 werden direkt oder bedarfsweise - wie in Fig. 1 dargestellt - über einen Vorverstärker 12 an die Meßeinrichtung 10 ausgegeben. Der Vorverstärker 12 kann beispielsweise einer elektronischen Kompensation der Meßspulen 11 und/oder einer Impedanzanpassung o. dgl. dienen.

Die Meßeinrichtung 10 ermittelt bzw. mißt die Amplitude und/oder Phase der Schwingung der Partikel 3 im Fluid 2. Insbesondere zur Bestimmung der Phase, genauer gesagt der Phasenverschiebung gegenüber dem auf die Partikel 3 wirkenden Anregungsfeld bzw. Magnetfeld 6, kann die Steuereinrichtung 7 bedarfsweise ein entsprechendes Synchronisations- bzw. Referenzsignal an die Meßeinrichtung 10 übertragen, wie beim Darstellungsbeispiel durch eine entsprechende Verbindung in Fig. 1 angedeutet.

Die Meßeinrichtung 10 arbeitet nach dem sogenannten Lock-In-Verfahren und weist einen sogenannten Lock-In-Verstärker auf.

Die Meßeinrichtung 10 kann zusätzlich zu dem bevorzugten Lock-In-Verfahren auch auf sonstige Weise die Amplitude und/oder Phase der Schwingung der Partikel 3 im Fluid 2 messen.

Die Amplitude kann beispielsweise nach entsprechender Kalibrierung absolut gemessen werden. Sie kann aber auch relativ gemessen werden, beispielsweise der zeitliche Verlauf bzw. die zeitliche Änderung der Amplitude über die Zeit. Dieser zeitliche Verlauf stellt beispielsweise bei der Gerinnung von Blut als Fluid 2 ein Maß für das Fortschreiten der Gerinnung dar.

Die Amplitude und Phase der Partikelschwingung hängen von der Dämpfung und damit von der Viskosität ab. Aus der gemessenen Amplitude und/oder Phase wird die Viskosität und/oder eine damit zusammenhängende Größe des Fluids 2 und/oder der Partikel 3 bestimmt. Dies erfolgt insbesondere mittels einer Auswerteeinrichtung 13, insbesondere einem Computer oder dergleichen.

Die Viskosität bzw. die damit zusammenhängende Größe ist vorzugsweise anzeigbar und/oder über eine hier nicht dargestellte Schnittstelle, beispielsweise zur Weiterverarbeitung, ausgebbar.

Die Sensoreinrichtung 9 kann anstelle von Meßspulen 11 zusätzlich oder alternativ einen sonstigen magnetischen und/oder elektrisch arbeitenden Sensor zur Detektion der Schwingung der Partikel 3 aufweisen. Beispielsweise kann als Sensor ein GMR (Giant Magneto-Resistance), TMR (Tunnel Magneto-Resistance), AMR (Anisotropic Magneto-Resistance) bzw. ein Magnetowiderstand, eine Magnetoimpedanz, ein Hall-Sensor oder dergleichen eingesetzt werden. Insbesondere wird die Schwingung der Partikel 3 aufgrund magnetischer Effekte, Beeinflussungen bzw. Eigenschaften erfaßt. Beispielsweise ist bei sogenannten XMR-Sensoren, wie GMR-Sensoren, oder bei Hall-Sensoren durch Messung des magnetischen Streufelds eine Bestimmung der Schwingungsamplitude möglich. Jedoch kann der Sensor ggf. auch akustisch, kapazitiv oder induktiv arbeiten.

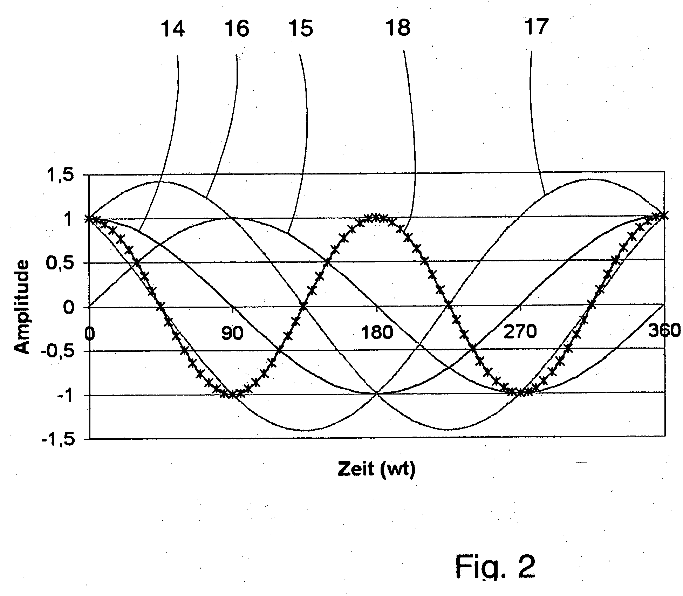

Die beiden Spulen 5 erzeugen jeweils periodische, sinusförmige Magnetfelder, die durch die Steuereinrichtung 7 um 90° phasenversetzt sind. Das nur schematische Diagramm gemäß Fig. 2 gibt nur eine grobe Näherung für die Mitte zwischen den Spulen 5 wieder und zeigt den zeitlichen Verlauf der beiden von den Spulen 5 erzeugten Magnetfelder, die als Linien 14 und 15 mit einer auf den Wert 1 normierten Amplitude dargestellt sind.

Die beiden von den Spulen 5 erzeugten Magnetfelder addieren sich zu dem auf die Partikel 3 wirkenden, zeitlich variierenden und inhomogenen Magnetfeld 6, das in Fig. 2 als Linie 16 dargestellt ist.

Zu beachten ist, daß die Partikel 3, zumindest wenn sie superparamagnetisch sind, immer eine Kraft in Richtung des stärkeren Magnetfelds unabhängig von dessen Polarisierung, also in Richtung des räumlichen Feldgradienten erfahren. Dies läßt sich derart erklären, daß zumindest superparamagnetische Partikel 3 ihre magnetischen Momente immer derart in Richtung des Magnetfelds 6 ausrichten, daß sie zu der das Magnetfeld 6 erzeugenden (stärkeren) Spule 5 gezogen werden.

In Fig. 2 zeigt Linie 17 die räumliche Ableitung des Magnetfelds 6 zwischen den beiden Spulen 5, wobei die Veränderung der Lage der Partikel 3 durch ihre Schwingung - ausgehend von einer nur sehr geringen Schwingungsamplitude - vernachlässigt wurde. Die Multiplikation der beiden Kurven 16 und 17 ergibt die Linie 18, die ein Maß für die auf die Partikel 13 wirkende, vom Magnetfeld 6 erzeugte Kraft darstellt. Hierbei ist zu bemerken, daß der Verlauf der auf die Partikel 3 wirkenden Kraft die doppelte Frequenz des Magnetfelds 6 aufweist. Dementsprechend schwingen die Partikel 3 bei der ersten Ausführungsform mit der doppelten Anregungsfrequenz.

Aufgrund der doppelten Schwingungsfrequenz der Partikel 3 gegenüber dem zeitlich variierenden bzw. wechselnden Magnetfeld 6 werden - insbesondere bei dem Lock-In-Verfahren - Störsignale, die insbesondere von (unterschiedlichen) magnetischen Suszeptibilitäten herrühren, zumindest weitgehend ausgeblendet, da diese nicht die doppelte Frequenz, sondern nur die Anregungsfrequenz zeigen. Als Meßsignale der Meßeinrichtung 10 - insbesondere des die doppelte Anregungsfrequenz auswertenden Lock-In-Verstärkers - ergeben sich Signale der Schwingung der Partikel 3 und Signale basierend auf den Suszeptibilitäten der Partikel 3.

Die Messung (Meßsignal ∝ Amplitude Frequenz / Abstand⁴ der Partikelschwingung) der Amplitude und/oder der Phase der Partikelschwingung ist somit verhältnismäßig einfach. Das Ergebnis stellt ein Maß für die Dämpfung der Partikelschwingung im Fluid 2 und damit für die Viskosität im genannten Sinne dar. Bei entsprechender Kalibrierung und/oder beispielsweise einer simultan verlaufenden Vergleichsmessung kann dementsprechend aus der Amplitude bzw. Phase die Viskosität und/oder eine damit zusammenhängende Größe des Fluids 2 bzw. der Partikel 3 im erläuterten Sinne bestimmt werden.

Bei der ersten Ausführungsform wirkt im wesentlichen nur das vom Magnetfeld 6 gebildete Wechselfeld auf die magnetischen Partikel 3. Dies ist insbesondere dann realisierbar, wenn die magnetischen Partikel 3 im Fluid 2 ausreichend gleichmäßig und/oder in ausreichender Konzentration im relevanten Meßbereich vorhanden sind und/oder wenn die Partikel 3 von dem Magnetfeld 6 aufgrund dessen Inhomogenität in ausrechendem Maß im Meßbereich konzentriert oder gehalten werden.

Wenn hingegen zusätzlich ein insbesondere stationäres Magnetfeld auf die Partikel 3 - beispielsweise zur Konzentration im Meßbereich und/oder definierten Ausrichtung der magnetischen Momente - wirkt, werden die magnetischen Momente der Partikel 3 beeinflußt und gegebenenfalls festgelegt, so daß die bei der ersten Ausführungsform vorgesehene Verdoppelung der Frequenz der Schwingung der Partikel 3 gegenüber der Frequenz des zeitlich variierenden, alternierenden Magnetfelds 6 nicht auftritt, weil sich die magnetischen Momente nicht frei zum Magnetfeld 6 ausrichten können.

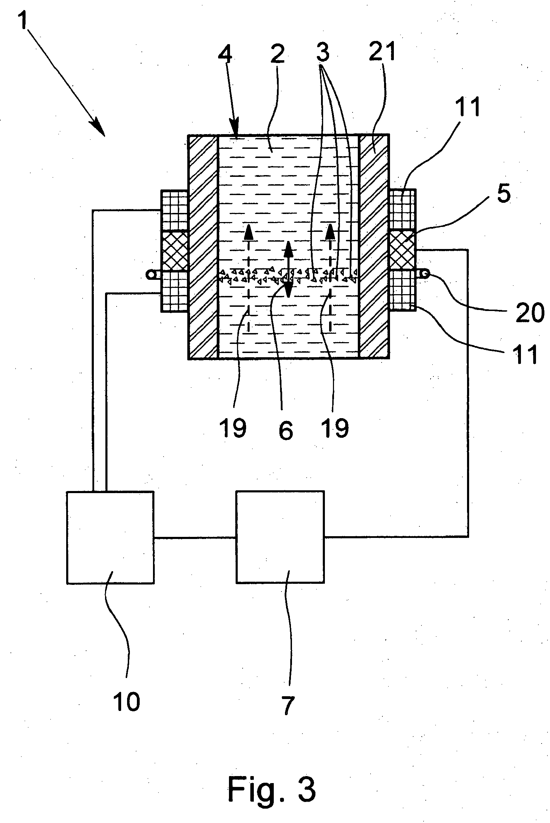

Fig. 3 zeigt ein Beispiel, welches nicht Teil der Erfindung ist. Nachfolgend werden lediglich wesentliche Unterschiede gegenüber der ersten Ausführungsform erläutert. Ansonsten ergeben sich die gleichen oder zumindest ähnliche Vorteile und Eigenschaften.

Es ist nur eine Spule 5 zur Erzeugung des zeitlich variierenden Magnetfelds 6 vorgesehen.

Jedoch können auch zwei Spulen 5 vorgesehen sein, die vorzugsweise als antiparallel geschaltete Helmholtz-Spulen ausgebildet sind. Die Spulen 5 sind dann vorzugsweise entgegengesetzt gewickelt, so daß bei einer sinusförmigen Anregung immer der Nordpol bzw. der Südpol der Spulen 5 zueinander gerichtet sind. Zur Erzeugung des zeitlich variierenden Magnetfelds 6 genügt aufgrund der entgegengesetzten Wicklungen der Spulen 5 nur ein Oszillator oder Funktionsgenerator.

Die Detektion der Partikelschwingung erfolgt auch bei der zweiten Ausführungsform durch die Meßspulen 11. Diese sind vorzugsweise gegenseitig kompensiert, so daß das Magnetfeld 6 der Spule 5 im Idealfall kein Signal in der Meßeinrichtung 10, insbesondere in dessen Lock-In-Verstärker oder dergleichen, erzeugt.

Bei der zweiten Ausführungsform sind vorzugsweise nur ein oder zwei Meßspulen 11 vorgesehen. Die Meßspulen 11 sind vorzugsweise symmetrisch zur Spule 5 zur Kompensation des Wechselfelds 6 angeordnet, wobei die Meßspulen 11 die Meßkammer 4 radial umgeben.

An Stelle einer Meßspule 11 oder der gezeigten zwei Meßspulen 11 können auch wieder sonstige Sensoren zur Erfassung der Partikelschwingung eingesetzt werden.

Zusätzlich oder alternativ zum Vorverstärker 12 kann eine Wechselfeld-Impedanzbrücke zur Gegenkopplung in den Meßkreis geschaltet sein, um die Meßempfindlichkeit zu erhöhen.

Bei der zweiten Ausführungsform wird dem inhomogenen magnetischen Wechselfeld 6 der Spule 5 vorzugsweise ein stärkeres, zeitlich konstantes Magnetfeld 19 überlagert. Dieses Magnetfeld 19 wird entweder durch einen Elektromagneten - bei der Darstellung gemäß Fig. 3 durch eine Spule 20 mit nur einer Wicklung oder mehreren Wicklungen - oder durch einen nicht dargestellten Permanentmagneten bzw. Ringmagneten erzeugt.

Das stärkere Magnetfeld 19 ist in der Ebene der Spule 20 am stärksten und dient einer Fokussierung der Partikel 3 in dieser Ebene bzw. im Bereich der Spule 20 oder eines alternativ oder zusätzlich eingesetzten Magneten. Um zu verhindern, daß die Partikel 3 von der Spule 20 und ggf. von der Spule 5 aus der Mitte der Meßkammer 4 zur Wandung der Meßkammer 4 hin nach außen gezogen werden, ist der Meßkammer 4 eine diamagnetische Abschirmung 21 zugeordnet, die zwischen den Partikeln 3 und den Spulen 5, 20 angeordnet ist.

Die Abschirmung 21 ist beim Darstellungsbeispiel insbesondere hohlzylindrisch ausgebildet. Sie kann die Meßkammer 4 ggf. auch vollständig umgeben. Bedarfsweise kann die diamagnetische Abschirmung 21 auch unmittelbar von der Wandung der Meßkammer 4 gebildet sein bzw. die Meßkammer 4 bilden.

Die diamagnetische Abschirmung 21 bewirkt, daß die Partikel 3 bei Annäherung eine repulsive Kraft erfahren, also von der Abschirmung 21 abgestoßen werden. So ist es möglich, die Partikel 3 in einer Gleichgewichtslage bzw. in einem gewissen Bereich zu fokussieren bzw. zu halten und außerdem in der genannten Weise in eine erzwungene Schwingung - beim Darstellungsbeispiel entsprechend dem Doppelpfeil 6 - zu versetzen. Die Vorrichtung 1 weist also ein Mittel auf, um die Partikel 3 insbesondere magnetisch in einem Raumbereich, vorzugsweise dem örtlichen Meßbereich innerhalb der Meßkammer 4, zu halten oder zu fokussieren. Dieses Mittel kann jedoch bedarfsweise auch - ggf. alternativ ausschließlich - durch das Magnetfeld 6 gebildet werden, wenn dieses derart, insbesondere in Abhängigkeit von der erfaßten Lage des zumindest einen Partikels 3, gesteuert oder geregelt wird, daß der Partikel 3 oder mehrere Partikel 3 in dem örtlichen Bereich, vorzugsweise dem Meßbereich, zumindest für eine für die Messung ausreichende Zeitdauer gehalten wird bzw. werden.

Aufgrund des Magnetfelds 19 wird die Richtung der magnetischen Momente der Partikel 3 im Raum, nämlich in Richtung des homogenen Magnetfelds 19, fixiert. Das schwächere magnetische Wechselfeld 6 erzeugt deshalb nur eine Schwingung der Partikel 3 mit der selben Frequenz. Die bei der ersten Ausführungsform angesprochene Frequenzverdopplung tritt hier also nicht auf.

Die insbesondere durch die Wirkung der magnetischen Suszeptibilitäten hervorgerufenen Störsignale werden mit der einfachen Anregungsfrequenz des Magnetfelds 6 moduliert. Da die Partikel 3 mit der selben Frequenz schwingen, kann die Schwingung der Partikel 3 im wesentlichen nur bzw. am leichtesten durch die Phasenverschiebung gegenüber der Anregungsfrequenz detektiert werden.

Die diamagnetische Abschirmung 21 ist bei der ersten Ausführungsform vorzugsweise ebenfalls vorgesehen, jedoch aus Vereinfachungsgründen weggelassen.

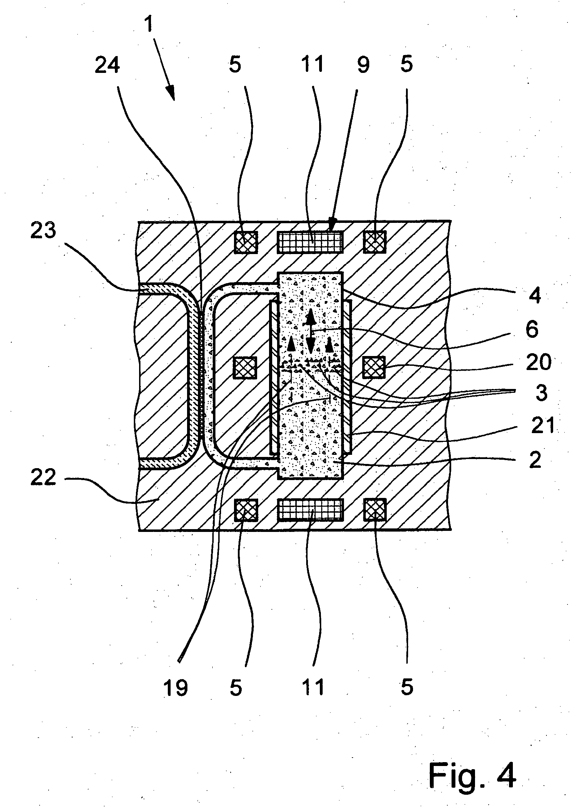

Fig. 4 zeigt in einem schematischen vertikalen Schnitt eine dritte Ausführungsform der vorschlagsgemäßen Vorrichtung 1. Nachfolgend werden lediglich wesentliche Unterschiede gegenüber der ersten und/oder zweiten Ausführungsform erläutert. Ansonsten ergeben sich zumindest im wesentlichen die gleichen Eigenschaften, Vorteile und technischen Realisierungsmöglichkeiten.

Bei der dritten Ausführungsform ist die Meßkammer 4 in einem vorzugsweise plattenförmigen Probenträger 22 ausgebildet. Der Probenträger 22 besteht vorzugsweise aus Kunststoff, in dem entsprechende Kavitäten ausgebildet sind. Beispielsweise kann es sich um einen Teststreifen o. dgl. handeln, der insbesondere für die chemische und/oder biologische Diagnostik bzw. mikrofluidische Untersuchungen eingesetzt wird.

Die Meßkammer 4 ist vorzugsweise mit senkrecht zur Plattenebene des Probenträgers 22 verlaufender Haupterstreckungsrichtung ausgebildet. Die Achsen der Spulen 5 und das zeitlich variierende Magnetfeld 6 verlaufen vorzugsweise in die gleiche Richtung wie die Meßkammer 4, also hier senkrecht zur Plattenebene des Probenträgers 22.

Die Schwingung der magnetischen Partikel 3 erfolgt daher vorzugsweise zumindest im wesentlichen senkrecht zur Plattenebene bzw. zu Flachseiten des Probenträgers 22.

Die Vorrichtung 1 bzw. der Probenträger 22 ist vorzugsweise derart ausgebildet, daß Blutplasma oder Blut 23 oder interstizielle Flüssigkeit, vorzugsweise selbsttätig durch Kapillarkräfte, aufnehmbar ist.

Gemäß einer nicht dargestellten Variante kann das Blut oder Blutplasma 23 unmittelbar als Fluid 2 dienen und beispielsweise direkt der Meßkammer 4 zuführbar sein. In diesem Fall enthält die Meßkammer 4 vorzugsweise die magnetischen Partikel 3 und insbesondere ein Gerinnungsmittel.

Nach Zuführung des Bluts bzw. Blutplasmas 23 werden die magnetischen Partikel 3, insbesondere durch die wirkenden Magnetfelder, im Blut bzw. Blutplasma 23 verteilt und/oder im Meßbereich - insbesondere durch das stationäre Magnetfeld 19 der Spule 20 - konzentriert, wobei durch die Schwingung der Partikel 3 gegebenenfalls auch eine gewisse Durchmischung oder Vermischung, insbesondere des nicht dargestellten Gerinnungsmittels mit dem Blut bzw. Blutplasma 23, erreicht werden kann.

Die Viskosität oder eine damit zusammenhängenden Größe wird wie bereits erläutert, insbesondere gemäß der ersten oder zweiten Ausführungsform, bestimmt und stellt insbesondere ein Maß für die Gerinnung (Koagulation) des Bluts bzw. Blutplasmas 23 in der Meßkammer 4 dar. Dementsprechend ist die Vorrichtung 1 bei dieser Variante zur unmittelbaren Messung bzw. Bestimmung der Koagulationsfähigkeit von Blut oder Blutplasma 23 ausgebildet.

Beim Darstellungsbeispiel gemäß Fig. 4 weist die Vorrichtung 1 bzw. der Probenkörper 22 zusätzlich eine Membran 24 auf, die für Glucose durchlässig ist. Insbesondere kann die Vorrichtung 1 dann ggf. auch interstizielle Flüssigkeit anstatt Blut oder Blutplasma 23 aufnehmen. Das Fluid 2 ist für Glucose sensitiv ausgebildet, und zwar dahingehend, daß sich die Viskosität des Fluids 2 in Abhängigkeit vom Glucosegehalt verändert. Beispielsweise enthält das Fluid 2 hochmolekulares Dextran, Concanavalin A als Affinitätsrezeptor für Glucose, Modifizierungen davon und/oder sonstige zuckerbindende Moleküle.

Das über die Membran 24 mit dem Blut oder Blutplasma 23 oder interstizieller Flüssigkeit in Glucoseaustausch stehende Fluid 2 kann beispielsweise mittels einer nicht dargestellten Pumpe oder Einrichtung in die Meßkammer 4 gepumpt oder durch diese und vorbei an der Membran 24 zirkuliert werden.

In der Meßkammer 4 wird die Viskosität oder eine damit zusammenhängende Größe des Fluids 2, insbesondere entsprechend der ersten oder zweiten Ausführungsform, und daraus der Glucosegehalt bestimmt.

Bedarfsweise kann das für Glucose sensitive Fluid 2 während der Messung der Amplitude und/oder Phase der Schwingung der magnetischen Partikel 3 weiterhin zugeführt oder zirkuliert werden. Bei einer einheitlichen bzw. zumindest im wesentlichen homogenen und konstanten Strömung des Fluids 2 durch die Meßkammer 4 kann bedarfsweise auch nur eine Spule 5 zur Erzeugung des zeitlich variierenden Magnetfelds 6 eingesetzt werden, so daß die magnetischen Partikel 3 durch Überlagerung der Strömung und der auf sie wirkenden magnetischen Kraft eine Schwingung in der Meßkammer 4 bzw. innerhalb des gewünschten Meßbereichs ausrühren, um die Viskosität und daraus den Glucosegehalt zu bestimmen.

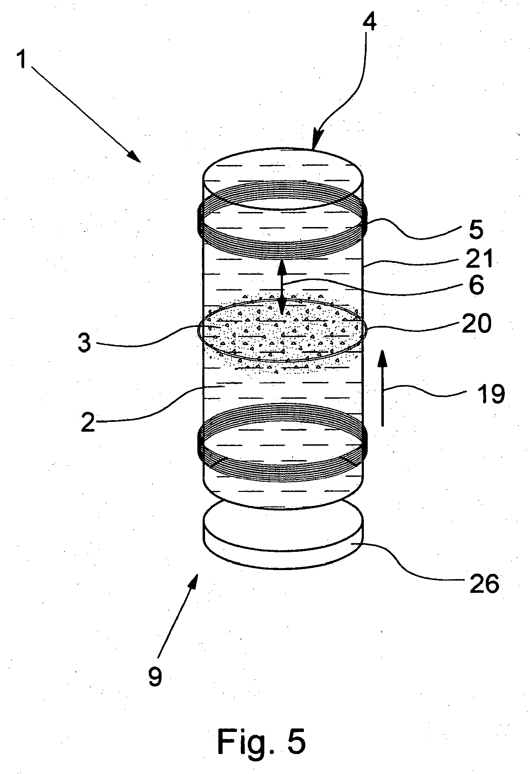

Fig. 5 zeigt in einer sehr schematischen Darstellung eine vierte Ausführungsform der vorschlagsgemäßen Vorrichtung 1, wobei nachfolgend lediglich wesentliche Unterschiede gegenüber den bereits erläuterten Ausführungsformen hervorgehoben werden. Ansonsten ergeben sich die gleichen oder zumindest ähnliche Vorteile und Eigenschaften.

Die Spulen 5 zur Erzeugung des zeitlich variierenden Magnetfelds 6 umgeben Endbereiche oder sind anschließend an die Enden der Meßkammer 4 angeordnet.

Zur Überlagerung des variierenden Magnetfelds 6 mit dem stärkeren, zeitlich konstanten Magnetfeld 19 ist wieder die Spule 20 vorgesehen.

Das statische Magnetfeld 19 bildet also wiederum - vorzugsweise in einem Zentralbereich und/oder zwischen den Spulen 5 - eine Inhomogenität bzw. Senke für die Partikel 3 bildet, so daß diese in diesem Bereich konzentriert bzw. dort gehalten werden. Die zusätzliche Spule 20 ist hierzu in axialer Richtung insbesondere sehr schmal bauend ausgebildet und gegebenenfalls nur durch eine einzige Windung gebildet. Das von der Spule 20 erzeugte Magnetfeld 19 ist ausreichend stark, um die Partikel 3 in ihre Spulenebene zu ziehen.

Das variierende Magnetfeld 6 bewirkt dann das Schwingen der Partikel 3 um die genannte Ausgangs- bzw. Ruhelage.

Die diamagnetische Abschirmung 21 ist bei der vierten Ausführungsform unmittelbar durch die Wandung der Meßkammer 4 gebildet.

Bei der vierten Ausführungsform weist die Sensoreinrichtung 9 anstelle der Meßspulen 11 einen GMR 26 als Sensor zur Detektion der Partikelschwingung auf. Insbesondere genügt ein einziger Sensor bzw. GMR 26 zur Detektion der Partikelschwingung, da dessen Widerstand bzw. Meßsignal stark in Abhängigkeit vom Abstand der Partikel 3 zum GMR 26 und damit korelliert zu der Partikelschwingung variiert. Eine Auswertung kann dann wie bei den bereits beschriebenen Ausführungsformen oder auf sonstige geeignete Weise erfolgen.

Gemäß einer bevorzugten Ausführungsvariante wird die Vorrichtung 1 zumindest im Bereich oder bei der Resonanzfrequenz der Partikel 3 betrieben. So wird eine verhältnismäßig große Amplitude der Partikel 3 bei verhältnismäßig geringem Leistungsbedarf erreicht, was einer genauen Messung von Amplitude und/oder Phase zuträglich ist.

Gemäß einer besonders bevorzugten Ausführungsvariante wird die Frequenz des variierenden Magnetfelds 6 permanent variiert und/oder vorzugsweise selbsttätig auf die Resonanzfrequenz oder eine Frequenz mit einer Mindestoder Maximalamplitude der Partikel 3 gesteuert oder geregelt.

Durch Variation der Frequenz des variierenden Magnetfelds 6 können die Amplitude und die Phase zumindest relativ bestimmt sowie Änderungen dieser Größen erfaßt werden. Hieraus können dann Änderungen der Viskosität und/oder der Partikel-Eigenschaften bestimmt werden.

Vorzugsweise werden aus der Resonanzfrequenz und/oder der Resonanzkurve - also der Abhängigkeit der Amplitude und/oder Phase der Partikelschwingung von der Frequenz des variierenden Magnetfelds 6 - die Größe und ggf. sonstige Eigenschaften der Partikel 3 bestimmt. Dies ist insbesondere bei bekannten Eigenschaften des Fluids 2 und/oder entsprechender Kalibrierung möglich.

Alternativ oder zusätzlich kann durch Messung der Amplitude und/oder Phase der Partikelschwingung und/oder Bestimmung der Resonanzkurve die Beweglichkeit der Partikel 3 im Fluid 2 und dadurch beispielsweise das Anlagern oder Ablösen von Atomen und Molekülen an die Partikel 3 bzw. von den Partikeln 3 qualitativ und gegebenenfalls quantitativ festgestellt bzw. bestimmt werden.

Gemäß einer weiteren Ausführungsvariante werden Partikel 3 unterschiedlicher Größe eingesetzt, insbesondere zumindest zwei Größen von Partikeln 3. Vorzugsweise wird dann wahlweise oder nacheinander im Bereich der unterschiedlichen Resonanzfrequenzen für die beiden Partikelgrößen die Messung von Amplitude und/oder Phase der Partikelschwingung durchgeführt.

Aus dem Vorgenannten ergibt sich, daß das vorschlagsgemäße Verfahren und die vorschlagsgemäße Vorrichtung 1 universell zur Messung der Viskosität oder einer damit zusammenhängenden Größe des Fluids 2, insbesondere einer Flüssigkeit bzw. der Partikel 3, geeignet sind, wobei insbesondere auch eine Bestimmung bzw. Erfassung der Koagulationsfähigkeit von Blut oder Blutplasma 23 oder die Erfassung eines Glucosegehalts ermöglicht wird. Das vorschlagsgemäße Verfahren und die vorschlagsgemäße Vorrichtung 1 sind insbesondere zum Einsatz in mikrofluidischen Systemen geeignet.

Wie bereits eingangs erläutert ist der Begriff "Viskosität" bei der vorliegenden Erfindung in einem engeren Sinne als innere Reibung bzw. Möglichkeit des Fluids 2, Spannungen bei Verformung aufzunehmen, zu verstehen. In einem weiteren Sinn ist unter "Viskosität" eine Veränderung von Eigenschaften des gegebenenfalls inhomogenen Fluids 2, insbesondere durch Koagulation oder Quellen oder Dissoziation von Bestandteilen oder dergleichen, und/oder eine Veränderung der Strömungseigenschaften oder sonstiger Eigenschaften der Partikel 3 im Fluid 2, beispielsweise durch Anlagerung von Atomen oder Molekülen an den Partikeln 3 oder Ablösen davon - wie das Bilden oder Auflösen von Komplexen - oder dergleichen, zu verstehen. Das vorschlagsgemäße Verfahren und die vorschlagsgemäße Vorrichtung 1 gestatten eine Bestimmung der Viskosität in diesem Sinne.

Alternativ oder zusätzlich werden mit dem vorschlagsgemäßen Verfahren und der vorschlagsgemäßen Vorrichtung 1 beispielsweise die Bestimmung der Reynolds-Zahl und/oder Strouhal-Zahl oder dergleichen ermöglicht.

Das vorschlagsgemäße Verfahren und die vorschlagsgemäße Vorrichtung 1 ist insbesondere auch zur Untersuchung bzw. Messung von inhomogenen Fluiden 2 geeignet.

Insbesondere ist die Vorrichtung 1 für die mikrofluidische Diagnostik ausgebildet. Vorzugsweise weist die Meßkammer 4 ein Volumen von höchstens 1 ml, vorzugsweise höchstens 500 µl, insbesondere höchstens 100 µl, oder von etwa 0,5 bis 20 µl auf.

## Patentansprüche

1. Verfahren zur Bestimmung der Viskosität oder einer damit zusammenhängenden Größe eines Fluids (2), insbesondere eines Mikrofluids,
wobei mindestens ein magnetischer Partikel (3) mit einem mittleren Durchmesser von 20 nm bis 1.000 µm im Fluid (2) mittels eines inhomogenen Magnetfelds (6) bewegt wird,
wobei der Partikel (3) durch zeitliche Variation des Magnetfelds (6) in Schwingung mit einer Amplitude von höchstens 1 mm versetzt und translatorisch hinund herbewegt wird,
wobei der Partikel (3) magnetisch in einem räumlichen Bereich innerhalb des Fluids (2) gehalten oder fokussiert wird, und
wobei die Amplitude und/oder Phase der Schwingung magnetisch gemessen wird bzw. werden und daraus die Viskosität oder die damit zusammenhängende Größe oder die Dämpfung der Partikelschwingung bestimmt wird,
**dadurch gekennzeichnet,**
**dass** das zeitlich variierende Magnetfeld (6) von zwei, in Schwingrichtung beabstandeten Spulen (5) erzeugt wird, und
**dass** die Magnetfelder der Spulen (5) sinusförmig, um 90° phasenversetzt, variiert werden und dass die Amplitude und/oder Phase der Schwingung mittels Lock In Technik gemessen wird.

2. Verfahren nach Anspruch 1, **dadurch gekennzeichnet, dass** das zeitlich variierende Magnetfeld (6) periodisch, insbesondere sinusförmig, variiert wird.

3. Verfahren nach Anspruch 1 oder 2, **dadurch gekennzeichnet, dass** der Partikel (3) mit der doppelten Frequenz der Magnetfelder der Spulen (5) schwingt.

4. Verfahren nach einem der voranstehenden Ansprüche, **dadurch gekennzeichnet, dass** der Partikel (3) mit der doppelten Frequenz des zeitlich variiertenden Magnetfelds (6) schwingt.

5. Verfahren nach einem der Ansprüche 1 oder 2, **dadurch gekennzeichnet, dass** das magnetische Moment des Partikels (3) mittels eines stationären Magnetfelds (19) in eine Richtung, vorzugsweise in eine Bewegungsrichtung der Schwingung, ausgerichtet wird.

6. Verfahren nach einem der voranstehenden Ansprüche, **dadurch gekennzeichnet, dass** der Partikel (3) mittels eines stationären Magnetfelds (19) in dem räumlichen Bereich im Fluid (2), insbesondere im Meßbereich und/oder im zeitlich variierenden Magnetfeld (6), fokussiert oder gehalten wird.

7. Verfahren nach Anspruch 5 oder 6, **dadurch gekennzeichnet, dass** das stationäre Magnetfeld (19) stärker als das zeitlich variierende Magnetfeld (6) ist.

8. Verfahren nach einem der voranstehenden Ansprüche, **dadurch gekennzeichnet, dass** der Partikel (3) mittels einer diamagnetischen Abschirmung (21) in dem räumlichen Bereich im Fluid (2), insbesondere in einem Meßbereich, stabilisiert wird.

9. Verfahren nach einem der voranstehenden Ansprüche, **dadurch gekennzeichnet, dass** zum Messen das Fluid (2) einer den Partikel (3) enthaltenden Meßkammer (4) zugeführt wird, insbesondere wobei mehrere Partikel (3) mittels des variierenden Magnetfelds (6) im Fluid (2) in Schwingung versetzt werden.

10. Verfahren nach einem der voranstehenden Ansprüche, **dadurch gekennzeichnet, dass** der Partikel (3) eine erzwungene Schwingung ausführt.

11. Verfahren nach einem der voranstehenden Ansprüche, **dadurch gekennzeichnet, dass** die Amplitude höchstens 0,5 mm, insbesondere 0,1 mm oder weniger, beträgt.

12. Verfahren nach einem der voranstehenden Ansprüche, **dadurch gekennzeichnet, dass** die Frequenz und/oder die Amplitude und/oder der zeitliche Verlauf des zeitlich variierenden Magnetfelds (6) derart gesteuert oder geregelt wird bzw. werden, dass die Amplitude der Schwingung des Partikels (3), zumindest für eine vorgebbare Zeitdauer, einen Mindestwert überschreitet.

13. Verfahren nach einem der voranstehenden Ansprüche, **dadurch gekennzeichnet, dass** die Schwingung des Partikels (3) mittels einer Sensoreinrichtung (9), die insbesondere mindestens eine Meßspule (11), einen Magnetowiderstand, eine Magnetoimpedanz, einen Hall-Sensor und/oder einen sonstigen Sensor aufweist, erfaßt wird.

14. Verfahren nach einem der voranstehenden Ansprüche, **dadurch gekennzeichnet, dass** die Amplitude und/oder Phase der Schwingung in Abhängigkeit von bzw. relativ zu dem variierenden Magnetfeld (6) bestimmt wird bzw. werden.

15. Verfahren nach einem der voranstehenden Ansprüche, **dadurch gekennzeichnet, dass** mehrere Partikel (3) im Fluid (2) verwendet bzw. in Schwingung versetzt werden.

16. Verfahren nach einem der voranstehenden Ansprüche, **dadurch gekennzeichnet, dass** superparamagnetische Partikel (3) und/oder Partikel (3) enthaltend Eisenoxid, insbesondere Magnetit oder sonstiges Ferrit, verwendet werden.

17. Verfahren nach einem der voranstehenden Ansprüche, **dadurch gekennzeichnet, dass** Partikel (3) mit einem mittleren Durchmesser von etwa 100 nm bis etwa 500 µm, insbesondere von etwa 0,5 µm bis etwa 100 µm, verwendet werden.

18. Verfahren nach einem der voranstehenden Ansprüche, **dadurch gekennzeichnet, dass** Partikel (3) mit einer Dichte zumindest im wesentlichen gleich der oder größer als die Dichte des Fluids (2) verwendet werden.

19. Verfahren nach einem der voranstehenden Ansprüche, **dadurch gekennzeichnet, dass** kernförmige, kugelförmige und/oder ellipsoidförmige Partikel (3) und/oder Partikel (3) mit einer Hülle, insbesondere aus Kunststoff, verwendet werden.

20. Verfahren nach einem der voranstehenden Ansprüche, **dadurch gekennzeichnet, dass** ein Fluidvolumen von weniger als 1 ml, vorzugsweise weniger als 100 µl, insbesondere weniger als etwa 20 µl, verwendet wird.

21. Vorrichtung (1) zur Bestimmung der Viskosität oder einer damit zusammenhängenden Größe eines Fluids (2), mit einer Meßkammer (4) zur Aufnahme des Fluids (2), mit mindestens einer Spule (5) zur Erzeugung eines zeitlich variierenden, inhomogenen Magnetfelds (6), so dass mindestens ein von dem Fluid (2) vollständig umgebener, magnetischer Partikel (3) mit einem mittleren Durchmesser von 20 nm bis 1.000 µm innerhalb des Fluids (2) mittels des variierenden Magnetfelds (6) in translatorische Schwingung mit einer Amplitude von höchstens 1 mm innerhalb des Fluids (2) versetzbar ist,
wobei der Partikel (3) innerhalb des Fluids (2) haltbar oder fokussierbar ist und dass die Vorrichtung (1) eine Sensoreinrichtung (9) und eine Messeinrichtung (10) zur magnetischen Erfassung der Partikelschwingung aufweist, deren Amplitude und/oder Phase zur Bestimmung der Viskosität oder der damit zusammenhängenden Größe oder der Dämpfung der Partikelschwingung meßbar ist bzw. sind,
**dadurch gekennzeichnet,**
**dass** die Vorrichtung (1) zwei, in Schwingrichtung beabstandete Spulen (5) zur Erzeugung des zeitlich variierenden Magnetfelds (6) und eine Einrichtung (7) zur Erzeugung von zwei, um 90° phasenversetzten Magnetfeldern aufweist und die Messeinrichtung einen Lock In Verstärker aufweist.

22. Vorrichtung nach Anspruch 21, **dadurch gekennzeichnet, dass** die Vorrichtung (1) als Probenträger (21), Mikrotiterplatte oder Teststreifen ausgebildet ist.

23. Vorrichtung nach Anspruch 21 oder 22, **dadurch gekennzeichnet, dass** das Aufnahmevolumen der Vorrichtung (1) oder einer Meßkammer (4) für Fluid (2) weniger als 1 ml, vorzugsweise weniger als 100 µl, insbesondere weniger als etwa 20 µl, beträgt.

24. Vorrichtung nach einem der Ansprüche 21 bis 23, **dadurch gekennzeichnet, dass** die Vorrichtung (1) derart ausgebildet ist, dass das zeitlich variierende Magnetfeld (6) periodisch, insbesondere sinusförmig, variierbar ist und/oder dass der Partikel (3) eine erzwungene Schwingung ausführt.

25. Vorrichtung nach einem der Ansprüche 21 bis 24, **dadurch gekennzeichnet, dass** die Spulen (5) als Helmholtz-Spulen ausgebildet sind.

26. Vorrichtung nach einem der Ansprüche 21 bis 25, **dadurch gekennzeichnet, dass** die Amplitude, Phasenverschiebung und/oder der Offset der Einrichtung (7) einstellbar ist bzw. sind.

27. Vorrichtung nach einem der Ansprüche 21 bis 26, **dadurch gekennzeichnet, dass** die Vorrichtung (1) eine Spule (20) oder einen Magneten zur Erzeugung eines stationären Magnetfelds (19) aufweist, um das magnetische Moment des Partikels (3) in eine Richtung, vorzugsweise in eine Bewegungsrichtung der Schwingung, auszurichten und/oder um den Partikel (3) in einem räumlichen Bereich im Fluid (2), insbesondere in einem Meßbereich, zu fokussieren oder zu halten.

28. Vorrichtung nach Anspruch 27, **dadurch gekennzeichnet, dass** das stationäre Magnetfeld (19) stärker als das zeitlich variierende Magnetfeld (6) ist.

29. Vorrichtung nach einem der Ansprüche 21 bis 28, **dadurch gekennzeichnet, dass** die Vorrichtung (1) eine diamagnetische Abschirmung (21) aufweist, um den Partikel (3), insbesondere mehrere Partikel (3), zu stabilisieren und/oder in der Meßkammer (4) bzw. in einem räumlichen Bereich im Fluid (2) zu fokussieren, insbesondere wobei die diamagnetische Abschirmung (21) zwischen dem Partikel (3) bzw. den Partikeln (3) und Spulen (5, 20) angeordnet ist.

30. Vorrichtung nach einem der Ansprüche 21 bis 29, **dadurch gekennzeichnet, dass** die Vorrichtung (1) eine vorzugsweise Partikel (3) enthaltende Meßkammer (4) zur Aufnahme von Fluid (2) aufweist.

31. Vorrichtung nach Anspruch 30, **dadurch gekennzeichnet, dass** die Meßkammer (4) in einem vorzugsweise plattenförmigen Probenträger (22) ausgebildet ist.

32. Vorrichtung nach Anspruch 31, **dadurch gekennzeichnet, dass** die Vorrichtung (1) derart ausgebildet ist, dass die Schwingung quer, insbesondere senkrecht, zu Flachseiten des Probenträgers (22) verläuft.

33. Vorrichtung nach Anspruch 31 oder 32, **dadurch gekennzeichnet, dass** Spulen (5) zur Erzeugung des zeitlich variierenden und/oder eines stationären Magnetfelds (6, 19) gegenüberliegend auf oder im Bereich von Flachseiten des Probenträgers (22) angeordnet sind.

34. Vorrichtung nach einem der Ansprüche 21 bis 33, **dadurch gekennzeichnet, dass** die Vorrichtung (1) derart ausgebildet ist, dass die Amplitude der Partikelschwingung höchstens 0,5 mm, insbesondere 0,1 mm oder weniger, beträgt.

35. Vorrichtung nach einem der Ansprüche 21 bis 34, **dadurch gekennzeichnet, dass** die Vorrichtung (1) eine Sensoreinrichtung (9) zur vorzugsweise unmittelbaren Erfassung der Partikelschwingung aufweist, vorzugsweise wobei die Sensoreinrichtung (9) mindestens eine Meßspule (11), einen Magnetowiderstand, eine Magnetoimpedanz, einen Hall-Sensor und/oder einen optischen Sensor aufweist.

36. Vorrichtung nach einem der Ansprüche 21 bis 35, **dadurch gekennzeichnet, dass** die Vorrichtung (1) mindestens einen superparamagnetischen Partikel (3), insbesondere in der Meßkammer (4), aufweist.

37. Vorrichtung nach einem der Ansprüche 21 bis 36, **dadurch gekennzeichnet, dass** die Vorrichtung (1) magnetische Partikel mit einer Größe von etwa 100 nm bis etwa 500 µm, insbesondere von etwa 0,5 µm bis etwa 100 µm, aufweist.

38. Vorrichtung nach einem der Ansprüche 21 bis 37, **dadurch gekennzeichnet, dass** die Vorrichtung (1) mindestens einen kornförmigen, kugelförmigen und/oder ellipsoidförmigen Partikel (3) und/oder magnetischen Partikel (3) enthaltend Eisenoxid, insbesondere Magnetit oder sonstiges Ferrit, aufweist.

39. Vorrichtung nach einem der Ansprüche 21 bis 38, **dadurch gekennzeichnet, dass** die Vorrichtung (1) magnetische Partikel (3) mit einer Hülle, insbesondere aus Kunststoff, aufweist.

40. Vorrichtung nach einem der voranstehenden Ansprüche 21 bis 39, **dadurch gekennzeichnet, dass** die Vorrichtung (1) zur Aufnahme von Blut oder Blutplasma (23) als Fluid (2), insbesondere selbsttätig durch Kapillarkräfte, ausgebildet ist.

41. Vorrichtung nach den Ansprüchen 30 und 40, **dadurch gekennzeichnet, dass** die Meßkammer (4) den oder die magnetischen Partikel (3) und ein Koagulationsmittel enthält, so dass nach Zuführung des Bluts oder Blutplasmas (23) die Koagulation durch Bestimmung der Viskosität meßbar bzw. erfaßbar ist.

42. Vorrichtung nach einem der Ansprüche 21 bis 41, **dadurch gekennzeichnet, dass** die Vorrichtung (1) zur Ausführung eines Verfahrens gemäß einem der Ansprüche 1 bis 20 ausgebildet ist.

43. Vorrichtung nach einem der Ansprüche 21 bis 39, **dadurch gekennzeichnet, dass** die Vorrichtung (1) eine Meßkammer (4) mit einem glucosesensitiven Fluid (2), das magnetische Partikel (3) enthält, sowie eine für Glucose durchlässige Membran (24) aufweist, wobei der Vorrichtung (1) Blut oder Blutplasma (23) oder interstizielle Flüssigkeit zuführbar ist, so dass das Blut oder Blutplasma (23) oder die interstizielle Flüssigkeit durch die Membran (24) mit dem glucosesensitiven Fluid (2) in Glucoseaustausch bringbar und durch Bestimmung der Viskosität des glucosesensitiven Fluids (2) der Glucosegehalt im Blut bzw. Blutplasma (23) oder in der interstiziellen Flüssigkeit meßbar bzw. erfaßbar ist.

44. Verwendung eines Verfahrens oder einer Vorrichtung (1) zur Bestimmung der Viskosität, um die Koagulationsfähigkeit von Blut zu nichttherapeutischen Zwecken zu bestimmen, wobei dem Blut mindestens ein magnetischer Partikel (3) und ggf. ein Koagulationgsmittel zugegeben werden, wobei mit einem Verfahren gemäß einem der Ansprüche 1 bis 20 oder einer Vorrichtung (1) gemäß einem der Ansprüche 21 bis 41 die Viskosität und daraus die Koagulationsfähigkeit des Bluts bestimmt werden.

45. Verwendung eines Verfahrens oder einer Vorrichtung (1) zur Bestimmung der Viskosität, um die Glucosekonzentration in einem glucosesensitiven Fluid (2) zu nichttherapeutischen Zwecken zu bestimmen, wobei dem Fluid (2) mindestens ein magnetischer Partikel (3) zugegeben ist, wobei mit einem Verfahren gemäß einem der Ansprüche 1 bis 20 oder einer Vorrichtung (1) gemäß einem der Ansprüche 21 bis 39 oder Anspruch 43 die Viskosität und daraus die Glucosekonzentration bestimmt werden.

46. Verwendung eines Verfahrens oder einer Vorrichtung (1) zur Bestimmung der Beweglichkeit von Partikeln (3) oder Dämpfung einer Bewegung von Partikeln (3) in einem Fluid (2), um eine Anlagerung, insbesondere von Atomen oder Molekülen, an die Partikel (3) im Fluid (2) oder eine Ablösung von den Partikeln (3) zu bestimmen, wobei dem Fluid (2) mindestens ein magnetischer Partikel (3) zugegeben wird, wobei mit einem Verfahren gemäß einem der Ansprüche 1 bis 20 oder einer Vorrichtung (1) gemäß einem der Ansprüche 21 bis 43 die Beweglichkeit, die Dämpfung und/oder deren Änderung des mindestens einen Partikels (3) im Fluid (2) und daraus die Anlagerung bestimmt werden.

## Claims

1. Method for determining the viscosity or a quantity related thereto of a fluid (2), in particular a micro-fluid.
wherein at least one magnetic particle (3) with a mean diameter of 20 nm to 1,000 µm is moved in the fluid (2) by means of an inhomogeneous magnetic field (6),
wherein the particle (3) is set by a temporal variation of the magnetic field (6) into oscillation with an amplitude of at most 1 mm and translationally reciprocated,
wherein the particle (3) is magnetically held or focused in a spatial area within the fluid (2), and
wherein the amplitude and/or phase of the oscillation is or are measured magnetically, and the viscosity or the quantity related thereto or the dampingof the particle oscillation is determined therefrom,
**characterized in**
**that** the time-varying magnetic field (6) is generated by two coils (5) spaced in the direction of oscillation, and
**that** the magnetic fields of the coils (6) are sinusoidally varied with a 90° phase shift, and
**that** the amplitude and/or phase of the oscillation is measured by means of lock-in technology.

2. Method according to claim 1, **characterized in that** the time-varying magnetic field (6) is periodically, in particular sinusoidally, varied.

3. Method according to claim 1 or 2, **characterized in that** the particle (3) oscillates at twice the frequency of the magnetic fields of the coils (5).

4. Method according to one of the preceding claims, **characterized in that** the particle (3) oscillates at twice the frequency of the time-varying magnetic field (6).

5. Method according to one of the claims 1 or 2, **characterized in that** the magnetic moment of the particle (3) is oriented in one direction, preferably in a direction of movement of the oscillation, by means of a stationary magnetic field (19).

6. Method according to one of the preceding claims, **characterized in that** the particle (3) is focused or held in the spatial region in the fluid (2), in particular in the measuring region and/or in the time-varying magnetic field (6), by means of a stationary magnetic field (19).

7. Method according to claim 5 or 6, **characterized in that** the stationary magnetic field (19) is stronger than the time-varying magnetic field (6).

8. Method according to one of the preceding claims, **characterized in that** the particle (3) is stabilized in the spatial region in the fluid (2), in particular in a measuring region, by means of diamagnetic shielding (21).

9. Method according one of the preceding claims, **characterized in that** for the measurement, the fluid (2) is fed to a measuring chamber (4) containing the particle (3), in particular wherein several particles (3) are oscillated in the fluid (2) by means of the varying magnetic field (6).

10. Method according to one of the preceding claims, **characterized in that** the particle (3) carries out a forced oscillation.

11. Method according to one of the preceding claims, **characterized in that** the amplitude is at most 0.5 mm, in particular 0.1 mm or less.

12. Method according to one of the preceding claims, **characterized in that** the frequency and/or the amplitude and/or the timing behavior of the time-varying magnetic field (6) is/are controlled or regulated such that the amplitude of the oscillation of the particle (3) exceeds, at least for a specifiable period of time, a minimum value.

13. Method according to one of the preceding claims, **characterized in that** the oscillation of the particle (3) is detected by means of a sensor device (9), which comprises, in particular, at least a measuring coil (11), a magneto-resistance, a magne-to-impedance, a Hall sensor and/or another sensor.

14. Method according to one of the preceding claims, **characterized in that** the amplitude and/or phase of the oscillation is/are determined as a function of or relative to the varying magnetic field (6).

15. Method according to one of the preceding claims, **characterized in that** several particles (3) are used or oscillated in the fluid (2).

16. Method according to one of the preceding claims, **characterized in that** super-paramagnetic particles (3) and/or particles (3) containing iron oxide, particularly magnetite or other ferrite, are used.

17. Method according to one of the preceding claims, **characterized in that** particles (3) with a mean diameter of about 100 nm to about 500 µm, in particular of about 0.5 µm to about 100 µm, are used.

18. Method according to one of the preceding claims, **characterized in that** particles (3) having a density at least substantially equal to or greater than the density of the fluid (2) are used.

19. Method according to one of the preceding claims, **characterized in that** grain-shaped, spherical and/or ellipsoidal particles (3) and/or particles (3) with a shell, in particular made of plastic, are used.

20. Method according to one of the preceding claims, **characterized in that** a fluid volume of less than 1 ml, preferably less than 100 µl, in particular less than about 20 µl, is used.

21. Device (1) for determining the viscosity or a quantity related thereto of a fluid (2), with a measuring chamber (4) to receive the fluid (2), with at least one coil (5) for generating a time-varying inhomogeneous magnetic field (6), so that at least one magnetic particle (3) completely surrounded by the fluid (2) with a mean diameter of 20 nm to 1,000 µm within the fluid (2) is setable into translational oscillation with an amplitude of at most 1 mm within the fluid (2) by means of the varying magnetic field (6),
wherein the particle (3) within the fluid (2) can be held or focused and that the device (1) has a sensor device (9) and a measuring device (10) for the magnetic detection of the particle oscillation, whose amplitude and/or phase is/are measurable to determine the viscosity or the quantity related thereto, or the damping of the particle oscillation,
**characterized in**
**that** the device (1) comprises two coils (5) spaced in the direction of oscillation for generating the time-varying magnetic field (6) and a device (7) for generating two 90° phase-shifted magnetic fields, and the measuring device comprises a lock-in amplifier.

22. Device according to claim 21, **characterized in that** the device (1) is configured as a sample carrier (21), a microtiter plate or a test strip.

23. Device according to claim 21 or 22, **characterized in that** the receiving volume of the device (1) or of a measuring chamber (4) for fluid (2) is less than 1 ml, preferably less than 100 µl, particularly less than about 20 µl.

24. Device according to one of the claims 21 to 23, **characterized in that** the device (1) is designed such that the time-varying magnetic field (6) can be periodically, in particular sinusoidally, varied and/or that the particle (3) carries out a forced oscillation.

25. Device according to one of the claims 21 to 24, **characterized in that** the coils (5) are formed as Helmholtz coils.

26. Device according to one of the claims 21 to 25, **characterized in that** the amplitude, phase shift and/or offset of the device (7) is/are adjustable.

27. Device according to one of the claims 21 to 26, **characterized in that** the device (1) comprises a coil (20) or a magnet for generating a stationary magnetic field (19) in order to orient the magnetic moment of the particle (3) in a direction, preferably in a direction of movement of the oscillation, and/or to focus or hold the particle (3) in a spatial region in the fluid (2), in particular in a measuring region.

28. Device according to claim 27, **characterized in that** the stationary magnetic field (19) is stronger than the time-varying magnetic field (6).

29. Device according to one of the claims 21 to 28, **characterized in that** the device (1) comprises a diamagnetic shielding (21) in order to stabilize the particle (3), in particular several particles (3), and/or to focus it in the measuring chamber (4) or in a spatial region in the fluid (2) in particular wherein the diamagnetic shielding (21) is arranged between the particle (3) or particles (3) and the coils (5, 20).

30. Device according to one of the claims 21 to 29, **characterized in that** the device (1) preferably comprises a measuring chamber (4), which contains particles (3), for receiving fluid (2).

31. Device according to claim 30, **characterized in that** the measuring chamber (4) is configured in a preferably plate-shaped sample carrier (22).

32. Device according to claim 31, **characterized in that** the device (1) is configured such that the oscillation runs transversely, in particular perpendicularly, to the flat sides of the sample carrier (22).

33. Device according to claim 31 or 32, **characterized in that** the coils (5) for generating the time-varying and/or a stationary magnetic field (6, 19) are arranged opposite to one another on or in the region of the flat sides of the sample carrier (22).

34. Device according to one of the claims 21 to 33, **characterized in that** the device (1) is configured such that the amplitude of the particle oscillation is at most 0.5 mm, particularly 0.1 mm or less.

35. Device according to one of the claims 21 to 34, **characterized in that** the device (1) comprises a sensor device (9) for preferably immediate detection of the particle oscillation, preferably wherein the sensor device (9) comprises at least a measuring coil (11), a magneto-resistance, a magneto-impedance, a Hall sensor and/or an optical sensor.

36. Device according to one of the claims 21 to 35, **characterized in that** the device (1) comprises at least one superparamagnetic particle (3), in particular in the measuring chamber (4).

37. Device according to one of the claims 21 to 36, **characterized in that** the device (1) comprises magnetic particles with a size of about 100 nm to about 500 µm, in particular of about 0.5 µm to about 100 µm.

38. Device according to one of the claims 21 to 37, **characterized in that** the device (1) comprises at least one grain-shaped, spherical and/or ellipsoidal particle (3) and/or a magnetic particle (3) containing iron oxide, particularly magnetite or other ferrite.

39. Device according to one of the claims 21 to 38, **characterized in that** the device (1) comprises magnetic particles (3) with a shell, in particular made of plastic.

40. Device according to one of the preceding claims 21 to 39, **characterized in that** the device (1) is configured to receive blood or blood plasma (23) as the fluid (2), in particular automatically through capillary forces.

41. Device according to the claims 30 and 40, **characterized in that** the measuring chamber (4) contains the magnetic particle (3) or particles (3) and a coagulant, so that after introduction of the blood or blood plasma (23), the coagulation is measurable or detectable by determining the viscosity.

42. Device according to one of the claims 21 to 41, **characterized in that** the device (1) is configured to implement a method according to one of the claims 1 to 20.

43. Device according to one of the claims 21 to 39, **characterized in that** the device (1) comprises a measuring chamber (4) with a glucose-sensitive fluid (2) which contains magnetic particles (3), as well as a membrane (24) permeable for glucose, wherein the device (1) can be fed with blood or blood plasma (23) or interstitial fluid, so that the blood or blood plasma (23) or the interstitial fluid can enter glucose exchange with the glucose-sensitive fluid (2) through the membrane (24) and the glucose content in the blood or blood plasma (23) or in the interstitial fluid is measurable or detectable by determination of the viscosity of the glucose-sensitive fluid (2).

44. Use of a method or a device (1) for determining the viscosity in order to determine the coagulability of blood for non-therapeutic purposes, wherein at least one magnetic particle (3) and optionally a coagulant are added to the blood, wherein the viscosity and therefrom the coagulability of the blood is determined with a method according to one of the claims 1 to 20 or a device (1) according to one of the claims, 21 to 41.

45. Use of a method or a device (1) for determining the viscosity in order to determine the glucose concentration in a glucose-sensitive fluid (2) for non-therapeutic purposes, wherein at least one magnetic particle (3) is added to the fluid (2), wherein the viscosity and therefrom the glucose concentration is determined with a method according to one of the claims 1 to 20 or a device (1) according to one of the claims 21 to 39 or claim 43.

46. Use of a method or a device (1) for determining the mobility of particles (3) or the damping of a movement of particles (3) in a fluid (2) in order to determine an attachment, particularly of atoms or molecules, to the particles (3) in the fluid (2), or a detachment from the particles (3), wherein at least one magnetic particle (3) is added to the fluid (2), wherein the mobility, the damping and/or their change of the at least one particle (3) in the fluid (2) and therefrom the attachment is determined with a method according to one of the claims 1 to 20 or a device (1) according to one of the claims 21 to 43.

## Revendications

1. Procédé pour la détermination de la viscosité ou d'une grandeur dépendant de celle-ci d'un fluide (2), en particulier d'un micro-fluide,
au moins une particule magnétique (3), avec un diamètre moyen de 20 nm à 1000 µm, étant déplacée dans le fluide (2) à l'aide d'un champ magnétique inhomogène (6),
la particule (3) étant mise en oscillation à l'aide d'une variation temporelle du champ magnétique (6) avec une amplitude de 1 mm maximum et déplacée en translation avec un mouvement de va-et-vient,
la particule (3) étant maintenue ou focalisée magnétiquement dans une zone spatiale à l'intérieur du fluide (2) et
l'amplitude et/ou la phase de l'oscillation étant mesurée magnétiquement et, à partir de celle-ci, la viscosité ou la grandeur dépendant de celle-ci ou l'amortissement de l'oscillation de la particule est déterminée,
**caractérisé en ce**
**que** le champ magnétique (6) variant dans le temps est généré par deux bobines (5) distantes dans la direction d'oscillation et
**que** les champs magnétiques des bobines (5) sont soumis à une variation sinusoïdale, avec un décalage de phase de 90° et
**que** l'amplitude et/ou la phase de l'oscillation est mesurée à l'aide d'une technique de lock-in.

2. Procédé selon la revendication 1, **caractérisé en ce que** le champ magnétique variable dans le temps (6) subit une variation périodique, en particulier sinusoïdale.

3. Procédé selon la revendication 1 ou 2, **caractérisé en ce que** la particule (3) oscille avec le double de la fréquence des champs magnétiques des bobines (5).

4. Procédé selon l'une des revendications précédentes, **caractérisé en ce que** la particule (3) oscille avec le double de la fréquence du champ magnétique variable dans le temps (6).

5. Procédé selon l'une des revendications 1 ou 2, **caractérisé en ce que** le moment magnétique de la particule (3) est orienté, au moyen d'un champ magnétique stationnaire (19) dans une direction, de préférence dans une direction de déplacement de l'oscillation.

6. Procédé selon l'une des revendications précédentes, **caractérisé en ce que** la particule (3) est focalisée ou maintenue, au moyen d'un champ magnétique stationnaire (19), dans la zone spatiale dans le fluide (2), en particulier dans la zone de mesure et/ou dans le champ magnétique variable dans le temps (6).

7. Procédé selon la revendication 5 ou 6, **caractérisé en ce que** le champ magnétique stationnaire (19) est supérieur au champ magnétique variable dans le temps (6).

8. Procédé selon l'une des revendications précédentes, **caractérisé en ce que** la particule (3) est stabilisée, au moyen d'un blindage diamagnétique (21), dans la zone spatiale dans le fluide (2), en particulier dans une zone de mesure.

9. Procédé selon l'une des revendications précédentes, **caractérisé en ce que**, pour la mesure, le fluide (2) est introduit dans une chambre de mesure (4) contenant la particule (3), en particulier plusieurs particules (3) étant mis en oscillation au moyen du champ magnétique variable (6) dans le fluide (2).

10. Procédé selon l'une des revendications précédentes, **caractérisé en ce que** la particule (3) effectue une oscillation forcée.

11. Procédé selon l'une des revendications précédentes, **caractérisé en ce que** l'amplitude est de 0,5 mm maximum, en particulier de 0,1 mm ou moins.

12. Procédé selon l'une des revendications précédentes, **caractérisé en ce que** la fréquence et/ou l'amplitude et/ou la variation dans le temps du champ magnétique variable dans le temps (6) est contrôlée ou régulée, de façon à ce que l'amplitude de l'oscillation de la particule (3) dépasse une valeur minimale au moins pour une durée spécifiable.

13. Procédé selon l'une des revendications précédentes, **caractérisé en ce que** l'oscillation de la particule (3) est mesurée au moyen d'un dispositif de capteur (9), qui comprend en particulier au moins une bobine de mesure (11), une résistance magnétique, une impédance magnétique, un capteur à effet Hall et/ou un autre capteur.

14. Procédé selon l'une des revendications précédentes, **caractérisé en ce que** l'amplitude et/ou la phase de l'oscillation est déterminée en fonction ou par rapport au champ magnétique variable dans le temps (6).

15. Procédé selon l'une des revendications précédentes, **caractérisé en ce que** plusieurs particules (3) sont utilisées dans le fluide (2) ou sont mises en oscillation.

16. Procédé selon l'une des revendications précédentes, **caractérisé en ce que** des particules super-paramagnétiques (3) et/ou des particules (3) contenant de l'oxyde de fer, en particulier de la magnétite ou une autre ferrite, sont utilisées.

17. Procédé selon l'une des revendications précédentes, **caractérisé en ce que** des particules (3) avec un diamètre moyen d'environ 100 nm à environ 500 µm, en particulier d'environ 0,5 µm à environ 100 µm, sont utilisées.

18. Procédé selon l'une des revendications précédentes, **caractérisé en ce que** des particules (3) avec une densité au moins globalement égale ou supérieure à la densité du fluide (2) sont utilisées.

19. Procédé selon l'une des revendications précédentes, **caractérisé en ce que** des particules (3) en forme de grains, de forme sphérique et/ou de forme ellipsoïde et/ou des particules (3) avec une coque, en particulier en matière plastique, sont utilisées.

20. Procédé selon l'une des revendications précédentes, **caractérisé en ce qu'**un volume de fluide inférieur à 1 ml, de préférence inférieur à 100 µl, plus particulièrement inférieur à environ 20 µl, est utilisé.

21. Dispositif (1) pour la détermination de la viscosité, ou de la grandeur dépendant de celle-ci, d'un fluide (2), avec une chambre de mesure (4) pour le logement du fluide (2), avec au moins une bobine (5) pour la génération d'un champ magnétique (6) inhomogène variable dans le temps, de façon à ce qu'au moins une particule magnétique (3), entourée entièrement par le fluide (2), avec un diamètre moyen de 20 nm à 1000 µm, est mise en oscillation de translation avec une amplitude de 1 mm maximum à l'intérieur du fluide (2) à l'aide du champ magnétique variable (6),
la particule (3) pouvant être maintenu ou focalisé à l'intérieur du fluide (2) et en ce que le dispositif (1) comprend un dispositif de capteur (9) et un dispositif de mesure (10) pour la mesure magnétique de l'oscillation de la particule, dont l'amplitude et/ou la phase peut être mesurée pour la détermination de la viscosité ou de la grandeur dépendant de celle-ci ou de l'amortissement de l'oscillation de la particule,
**caractérisé en ce**
**que** le dispositif (1) comprend deux bobines (5) distantes dans la direction d'oscillation pour la génération du champ magnétique variable dans le temps (6) et un dispositif (7) pour la génération de deux champs magnétiques déphasés de 90°, et le dispositif de mesure comprend un amplificateur de lock-in.

22. Dispositif selon la revendication 21, **caractérisé en ce que** le dispositif (1) est conçu comme un support d'échantillons (21), une plaque de microtitration ou une bande de test.

23. Dispositif selon la revendication 21 ou 22, **caractérisé en ce que** le volume du dispositif (1) ou d'une chambre de mesure (4) pour le fluide (2) est inférieur à 1ml, de préférence inférieur à 100 µl, en particulier inférieur à environ 20 µl.

24. Dispositif selon l'une des revendications 21 à 23, **caractérisé en ce que** le dispositif (1) est conçu de façon à ce que le champ magnétique varie dans le temps (6) est variable de manière périodique, en particulier de manière sinusoïdale, et/ou à ce que la particule (3) effectue une oscillation forcée.

25. Dispositif selon l'une des revendications 21 à 24, **caractérisé en ce que** les bobines (5) sont conçues comme des bobines de Helmholtz.

26. Dispositif selon l'une des revendications 21 à 25, **caractérisé en ce que** l'amplitude, le déphasage et/ou de décalage du dispositif (7) peut être réglé.

27. Dispositif selon l'une des revendications 21 à 26, **caractérisé en ce que** le dispositif (1) comprend une bobine (20) ou un aimant pour la génération d'un champ magnétique stationnaire (19), afin d'orienter le moment magnétique de la particule (3) dans une direction, de préférence dans une direction de déplacement de l'oscillation et/ou afin de focaliser ou de maintenir la particule (3) dans une zone spatiale dans le fluide (2), en particulier dans une zone de mesure.

28. Dispositif selon la revendication 27, **caractérisé en ce que** le champ magnétique stationnaire (19) est supérieur au champ magnétique variable dans le temps (6).

29. Dispositif selon l'une des revendications 21 à 28, **caractérisé en ce que** le dispositif (1) comprend un blindage diamagnétique (21) afin de stabiliser la particule (3), en particulier plusieurs particules (3) et/ou de la focaliser dans la chambre de mesure (4) ou dans une zone spatiale dans le fluide (2), en particulier le blindage diamagnétique (21) étant disposé entre la particule (3) ou les particules (3) et les bobines (5, 20).

30. Dispositif selon l'une des revendications 21 à 29, **caractérisé en ce que** le dispositif (1) comprend une chambre de mesure (4) pour le logement du fluide (2), de préférence contenant des particules (3).

31. Dispositif selon la revendication 30, **caractérisé en ce que** la chambre de mesure (4) est disposée dans un support d'échantillons (22), de préférence en forme de plaque.

32. Dispositif selon la revendication 31, **caractérisé en ce que** le dispositif (1) est conçu de façon à ce que l'oscillation s'étend transversalement, en particulier perpendiculairement, par rapport aux côtés plats du support d'échantillon (22).

33. Dispositif selon la revendication 31 ou 32, **caractérisé en ce que** des bobines (5) pour la génération du champ magnétique variable dans le temps et/ou d'un champ magnétique stationnaire (6, 19) sont disposées les unes en face des autres ou au niveau des côtés plats du support d'échantillon (22).

34. Dispositif selon l'une des revendications 21 à 33, **caractérisé en ce que** le dispositif (1) est conçu de façon à ce que l'amplitude de l'oscillation de la particule est de 0,5 mm maximum, en particulier de 0,1 mm ou moins.

35. Dispositif selon l'une des revendications 21 à 34, **caractérisé en ce que** le dispositif (1) comprend un dispositif de capteur (9) pour ta mesure, de préférence directe, de l'oscillation de la particule, de préférence le dispositif de capteur (9) comprenant au moins une bobine de mesure (11), une résistance magnétique, une impédance magnétique, un capteur à effet Hall et/ou un capteur optique.

36. Dispositif selon l'une des revendications 21 à 35, **caractérisé en ce que** le dispositif (1) comprend au moins une particule super-paramagnétique (3), en particulier dans la chambre de mesure (4).

37. Dispositif selon l'une des revendications 21 à 36, **caractérisé en ce que** le dispositif (1) comprend des particules magnétiques avec une taille d'environ 100 nm jusqu'à environ 500 µm, en particulier d'environ 0,5 µm à environ 100 µm.

38. Dispositif selon l'une des revendications 21 à 37, **caractérisé en ce que** le dispositif (1) comprend au moins une particule en forme de grain, de forme sphérique et/ou de forme ellipsoïde (3) et/ou une particule magnétique (3) contenant de l'oxyde de fer, en particulier de la magnétite ou une autre ferrite.

39. Dispositif selon l'une des revendications 21 à 38, **caractérisé en ce que** le dispositif (1) comprend des particules magnétiques (3) avec une coque, en particulier en matière plastique.

40. Dispositif selon l'une des revendications 21 à 39, **caractérisé en ce que** le dispositif (1) est conçu pour le logement de sang ou de plasma sanguin (23) en tant que fluide (2), en particulier automatiquement par des forces capillaires.

41. Dispositif selon les revendications 30 et 40, **caractérisé en ce que** la chambre de mesure (4) contient la ou les particules magnétiques (3) et un produit de coagulation de façon à ce que, après l'introduction du sang ou du plasma sanguin (23), la coagulation puisse être mesurée ou détectée par détermination de la viscosité.

42. Dispositif selon l'une des revendications 21 à 41, **caractérisé en ce que** le dispositif (1) est conçu pour la réalisation d'un procédé selon l'une des revendications 1 à 20.

43. Dispositif selon l'une des revendications 21 à 39, **caractérisé en ce que** le dispositif (1) comprend une chambre de mesure (4) avec un fluide (2) sensible au glucose, qui contient des particules magnétiques (3), ainsi qu'une membrane (24) perméable au glucose, le sang ou le plasma sanguin ou le liquide interstitiel pouvant être introduit dans le dispositif (1), de façon à ce que le sang ou le plasma sanguin (23) ou le liquide interstitiel puisse être mis, à travers la membrane (24) dans un échange de glucose avec le fluide (2) sensible au glucose, et la teneur en glucose dans le sang ou le plasma sanguin (23) ou dans le liquide interstitiel peut être mesurée ou détectée par détermination de la viscosité du fluide (2) sensible au glucose.

44. Utilisation d'un procédé ou d'un dispositif (1) pour la détermination de la viscosité afin de déterminer la capacité de coagulation du sang à des fins non thérapeutiques, au moins une particule magnétique (3) et, le cas échéant, un produit de coagulation, étant introduit dans le sang, un procédé selon l'une des revendications 1 à 20 ou un dispositif selon l'une des revendications 21 à 41 est utilisé pour déterminer la viscosité et donc la capacité de coagulation du sang.

45. Utilisation d'un procédé ou d'un dispositif (1) pour la détermination de la viscosité afin de déterminer la concentration en glucose dans un fluide (2) sensible au glucose, à des fins non thérapeutiques, au moins une particule magnétique (3) étant introduite dans le fluide (2), un procédé selon l'une des revendications 1 à 20 ou un dispositif (1) selon l'une des revendications 21 à 39 ou la revendication 43 est utilisé pour déterminer la viscosité et donc la concentration en glucose.

46. Utilisation d'un procédé ou d'un dispositif (1) pour la détermination de la capacité de déplacement des particules (3) ou de l'amortissement d'un déplacement des particules (3) dans un fluide (2), afin de déterminer un dépôt, en particulier d'atomes ou de molécules, sur les particules (3) dans le fluide (2) ou un détachement des particules (3), au moins une particule magnétique (3) étant introduite dans le fluide (2), un procédé selon l'une des revendications 1 à 20 ou un dispositif selon l'une des revendications 21 à 43 est utilisé pour déterminer la capacité de déplacement, l'amortissement et/ou la modification de l'au moins une particule (3) dans le fluide (2) et donc le dépôt.
